(19) 

Europäisches
Patentamt

European
Patent Office

Office européen
des brevets

(11)     **EP 4 036 764 A1**

(12)     **EUROPEAN PATENT APPLICATION**

(43) Date of publication:
      **03.08.2022 Bulletin 2022/31**

(21) Application number: **21213112.2**

(22) Date of filing: **08.12.2021**

(51) International Patent Classification (IPC):
      **G06F 17/16** (2006.01)      **G06N 20/00** (2019.01)

(52) Cooperative Patent Classification (CPC):
      **G06N 20/00; G06F 17/16**

(84) Designated Contracting States:
     **AL AT BE BG CH CY CZ DE DK EE ES FI FR GB
     GR HR HU IE IS IT LI LT LU LV MC MK MT NL NO
     PL PT RO RS SE SI SK SM TR**
     Designated Extension States:
     **BA ME**
     Designated Validation States:
     **KH MA MD TN**

(30) Priority:  **27.01.2021  JP 2021011225**

(71) Applicant: **FUJITSU LIMITED**
     **Kawasaki-shi, Kanagawa 211-8588 (JP)**

(72) Inventors:
     • **Tolmachev, Arseny**
       **Kawasaki-shi, Kanagawa, 211-8588 (JP)**
     • **Sakai, Akira**
       **Kawasaki-shi, Kanagawa, 211-8588 (JP)**

(74) Representative: **Hoffmann Eitle**
     **Patent- und Rechtsanwälte PartmbB**
     **Arabellastraße 30**
     **81925 München (DE)**

(54)     **MACHINE LEARNING PROGRAM, MACHINE LEARNING METHOD, AND MACHINE LEARNING APPARATUS**

(57)     A machine learning program that causes a computer to execute a process including specifying an axis of label mode and a plurality of axes of topology mode among a plurality of axes in a tensor format; selecting a first axis among the plurality axes of topology mode; calculating a core tensor by concatenating an element in a first element matrix corresponding to the axis of label mode to an element in a first intermediate tensor, by calculating a mode product of a second intermediate tensor and a second element matrix corresponding to another axis of topology mode other than the certain axis, by concatenating an element in a third element matrix corresponding to the certain axis and an element in the second element matrix to an element in a third intermediate tensor; and executing a machine learning by using the core tensor.

FIG. 6

**Description**

FIELD

The embodiments discussed herein are related to a machine learning program, a machine learning method, and a machine learning apparatus.

BACKGROUND

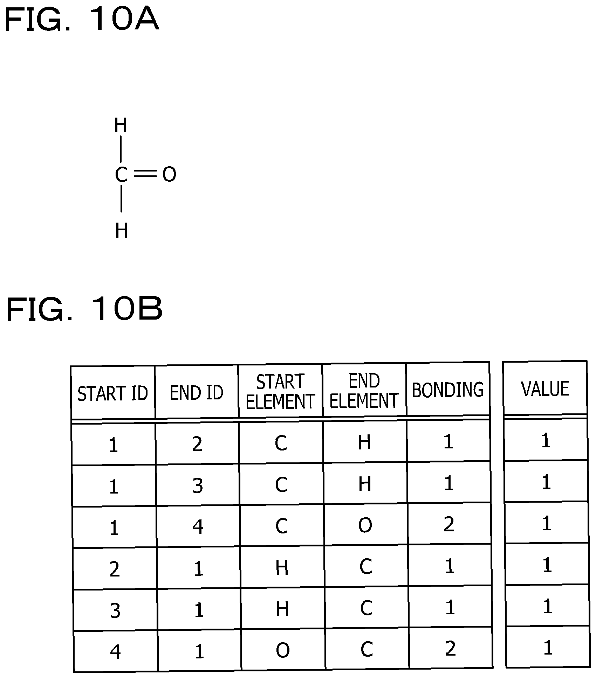

Tensor data may be used as training data for a system that generates a machine learning model by machine learning, and performs prediction, classification, and the like by using the generated machine learning model. The tensor data is expressed as an n-dimensional (n is a positive integer) array, and is also expressed by a graph, a table, or the like. FIGs. 10A and 10B are diagrams illustrating an example in which tensor data is expressed in a graph format and a table format. FIGs. 10A and 10B illustrate an example of a chemical formula. FIG. 10A illustrates a chemical formula of formaldehyde in a graph format, and FIG. 10B illustrates the chemical formula of formaldehyde in a table format.

In the table format, the chemical formula is expressed by 5 axes (columns) of a start ID, an end ID, a start element, an end element, and bonding, and values. The start ID is an index of a start point of linking. The end ID is an index of an end point of linking. The index may be freely assigned to the start point and the end point. The start element is a type of element at the start point. The end element is a type of element at the end point. The bonding is a type of linking. In the table format, only values having non-zero in the tensor data are expressed as rows. In FIG. 10B, only 6 values expressed by 6 rows are 1, and the other values of the tensor data are 0. The axis of the tensor is called a mode.

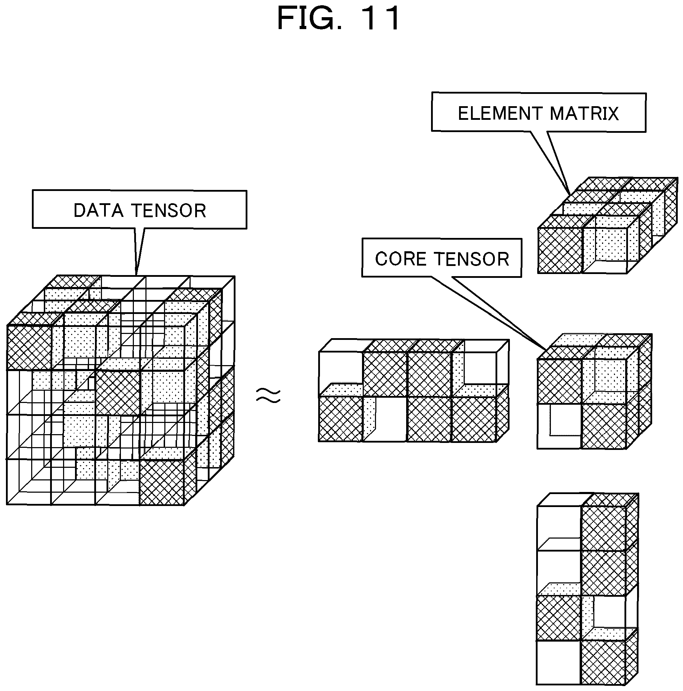

In machine learning, a data tensor is converted into a core tensor by tensor decomposition, and training is performed by using the core tensor. FIG. 11 is a diagram for explaining tensor decomposition. In FIG. 11, white cubes indicate elements of 0, and shaded cubes indicate elements having non-zero. As illustrated in FIG. 11, data tensor is tensor-decomposed into a core tensor and element matrices of the number of modes. In FIG. 11, since the data tensor has 3 stages and the number of modes is 3, the data tensor is tensor-decomposed into a core tensor and 3 element matrices.

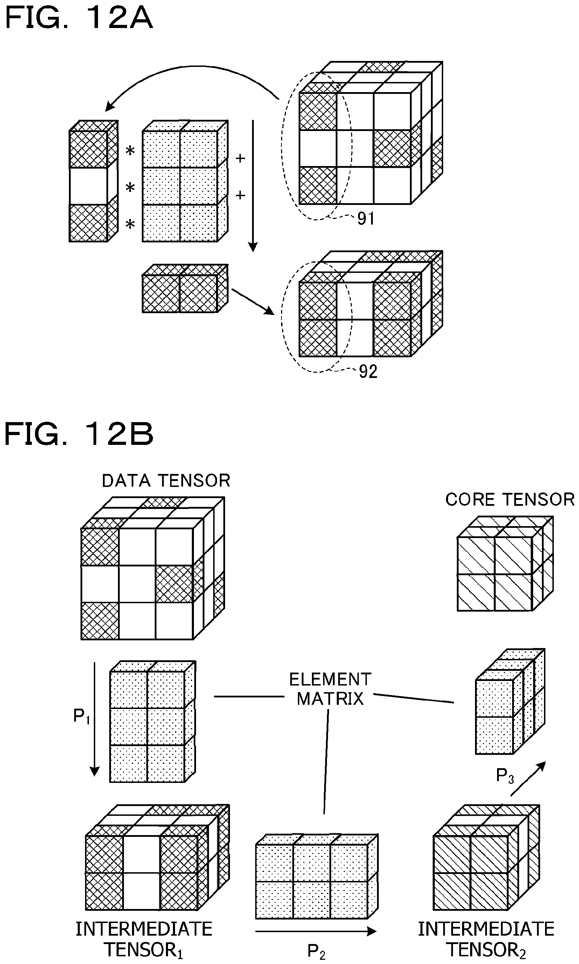

The core tensor is calculated by a mode product of the data tensor and the element matrix of each mode. FIGs. 12A and 12B are diagrams for explaining calculation of a core tensor by a mode product. FIG. 12A illustrates the mode product, and FIG. 12B illustrates the core tensor calculation. As illustrated in FIG. 12A, the number of elements of a mode is converted from the number of data tensors to the number of elements of the core tensor by the mode product. In the example in FIG. 12A, the number of elements of the mode in a vertical direction is converted from 3 to 2. When there is at least one element that is non-zero among the elements in a mode direction, all elements in the mode direction after the conversion are non-zero. In the example in FIG. 12A, since 3 elements 91 in the vertical direction include non-zero, 2 elements 92 after the conversion are non-zero. The number of elements of each mode in the core tensor is designated by a user.

As illustrated in FIG. 12B, the core tensor is calculated by sequentially calculating the mode products of the respective modes. In FIG. 12B, $P_i$ (i = 1 to 3) indicates a mode product corresponding to a mode i. As illustrated in FIG. 12B, an intermediate $tensor_1$ is calculated from a data tensor based on the mode product $P_1$, an intermediate $tensor_2$ is calculated from an intermediate $tensor_1$ by the mode product $P_2$, and a core tensor is calculated from an intermediate $tensor_2$ by the mode product $P_3$. An order of calculation of the mode products is arbitrary.

Assuming that a tensor having n stages is X, a core tensor is X^, and an element matrix of the mode i (i = 1 to n) is $W_i$,

$$X \in R^{I_1 \times I_2 \times \cdots \times I_n}$$

$$X^{\widehat{}} \in R^{J_1 \times J_2 \times \cdots \times J_n}$$

$$Wi \in R^{I_i \times J_i}$$

is established. R is a set of real numbers. $I_i$ is the number of elements of the mode i of X, and $J_i$ is the number of elements of the mode i of X^.

Regarding the mode product $P_k$ of the mode k, assuming that X is the following matrix X',

$$X' \in R^{I_k \times (I_1 \times \cdots \times I_{k-1} \times I_{k+1} \times \cdots \times I_n)}$$

the matrix product $X'' = (W_k)^T \times X'$ is calculated. Here,

$$X'' \in R^{J_k \times (I_1 \times \cdots \times I_{k-1} \times I_{k+1} \times \cdots \times I_n)}$$

is established. The mode of X" is rearranged to obtain an intermediate tensor $X^{intm}$.

$$X^{intm} \in R^{I_1 \times \cdots \times I_{k-1} \times J_k \times I_{k+1} \times \cdots \times I_n}$$

As the related art related to a data tensor, there is a tensor generation program capable of executing machine learning based on a ranking relationship of labels. The program causes a computer to execute a process of accepting an input of data having a graph structure including a plurality of nodes and attributes respectively set in the plurality of nodes. This program causes a computer to execute a process of generating tensor data which has dimensions respectively corresponding to the plurality of nodes and the respective attributes, and in which relationships between the plurality of nodes and the attributes and values corresponding to the relationships between the plurality of nodes are set. This program causes a computer to execute a process of setting a value in a range corresponding to a ranking relationship for each attribute included in the tensor data in a case where a ranking relationship between the attributes is machine-learned by using each attribute as a label.

Japanese Laid-open Patent Publication No. 2020-119101, U.S. Patent Application Publication No. 2019/0228304, and U.S. Patent Application Publication No. 2019/0325312 are disclosed as related art.

SUMMARY

[TECHNICAL PROBLEM]

Meanwhile, in the related art described above, there is a problem in that it is difficult to grasp a fine linking method (fine structure) of data in machine learning by using tensor data.

According to one aspect, there is provided a machine learning program, a machine learning method, and a machine learning apparatus that make it easier to grasp a fine linking method of data in machine learning.

[SOLUTION TO PROBLEM]

According to an aspect of the embodiments, a machine learning program that causes at least one computer to execute a process, the process includes specifying an axis of a label mode and a plurality of axes of a topology mode among a plurality of axes included in data in a tensor format; selecting a certain axis among the plurality axes of the topology mode; calculating a core tensor from the data in the tensor format via a plurality of intermediate tensors, by a first process of concatenating an element included in a first element matrix corresponding to the axis of the label mode to an element included in a first intermediate tensor among the plurality of intermediate tensors, by a second process of calculating a mode product of a second intermediate tensor among the plurality of intermediate tensors and a second element matrix corresponding to an axis among the plurality axes of the topology mode other than the certain axis, by a third process of concatenating an element included in a third element matrix corresponding to the certain axis and an element included in the second element matrix to an element included in a third intermediate tensor among the plurality of intermediate tensors; and executing a machine learning by using the core tensor as an input.

[ADVANTAGIOUS EFFECTS OF INVENTION]

It is possible to make it easy to grasp a fine linking method of data in machine learning.

BRIEF DESCRIPTION OF DRAWINGS

FIG. 1 is a diagram illustrating an example of a label mode and a topology mode;
FIG. 2 is a diagram for explaining a matrix product of a matrix having sparse non-zero elements and a matrix having dense non-zero elements;

FIG. 3 is a diagram for explaining a difference in mode product and concatenation;

FIG. 4 is a diagram for explaining an overview of an embodiment;

FIG. 5 is a diagram for explaining reassignment of an index;

FIG. 6 illustrates a functional configuration example of a machine learning apparatus according to the embodiment;

FIG. 7 is a diagram illustrating 5 modes;

FIG. 8 is a flowchart illustrating a procedure of a process by a core tensor calculation unit;

FIG. 9 is a diagram illustrating a hardware configuration of a computer that executes a machine learning program according to the embodiment;

FIGs. 10A and 10B are diagrams illustrating an example in which tensor data is expressed in a graph format and a table format;

FIG. 11 is a diagram for explaining tensor decomposition; and

FIGs. 12A and 12B are diagrams for explaining calculation of a core tensor by a mode product.

DESCRIPTION OF EMBODIMENTS

Hereinafter, a machine learning program, a machine learning method, and a machine learning apparatus according to embodiments will be described with reference to the drawings. In embodiments, components having the same functions are denoted by the same reference signs, thereby omitting redundant description thereof. The machine learning program, the machine learning method, and the machine learning apparatus described in the following examples are merely embodiments and are not intended to limit the embodiments. The embodiments below may be appropriately combined with each other within a scope without any contradiction.

[Embodiment]

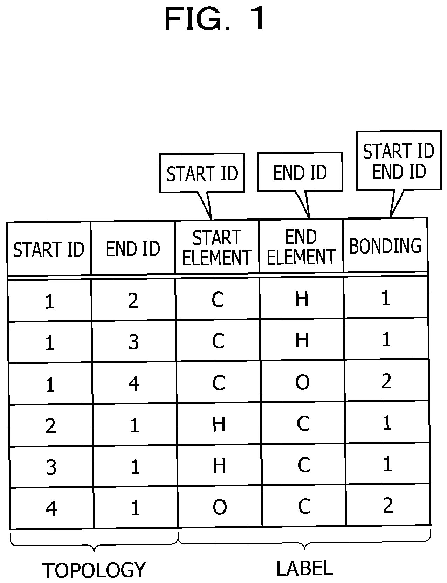

First, a label mode and a topology mode will be described. A machine learning apparatus according to the embodiment divides a mode into a topology mode and a label mode. The topology mode is a mode in which linking of data is expressed. The label mode is a mode in which an attribute is expressed. FIG. 1 is a diagram illustrating an example of the label mode and the topology mode. As illustrated in FIG. 1, a start ID and an end ID are topology modes, and a start element, an end element, and bonding are label modes. The label mode is associated with one or more of the topology modes, and a value of the label mode is determined by the associated topology mode.

For example, the start element is associated with the start ID, and a value of the start element is determined by the start ID. When the start ID is 1, the start element is C, and when the start ID is 2 or 3, the start element is H, and when the start ID is 4, the start element is O. The end element is associated with the end ID, and a value of end element is determined by the end ID. The end element is C when the end ID is 1, the end element is H when the end ID is 2 or 3, and the end element is O when the end ID is 4. The bonding is associated with the start ID and the end ID, and a value of the bonding is determined by the start ID and the end ID. When the start ID is 1 and the end ID is 2 or 3, the bonding is 1, and when the start ID is 1 and the end ID is 4, the bonding is 2.

The label mode is expressed as a one-hot vector. The one-hot vector is a vector in which only one element is 1 and the other elements are 0.

Next, conversion into concatenation of mode products by the machine learning apparatus according to the embodiment will be described. When many label modes are included in a data tensor, non-zero elements of the data tensor become sparse. On the other hand, an element matrix has dense non-zero elements. Calculation of the mode product is calculation of the matrix product $X'' = (W_k)^T \times X'$, and when many label modes are included in a data tensor, the number of non-zero elements of the intermediate tensor increases exponentially for each calculation of the mode product so as to calculate the matrix product of a matrix with sparse non-zero elements and a matrix with dense non-zero elements.

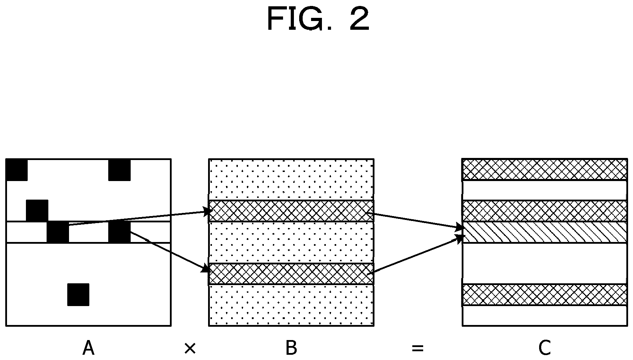

FIG. 2 is a diagram for explaining a matrix product of a matrix having sparse non-zero elements and a matrix having dense non-zero elements. In FIG. 2, A is a matrix with sparse non-zero elements, B is a matrix with dense non-zero elements, and C is a matrix product of A and B. In FIG. 2, black and shaded squares indicate non-zero elements, and white squares indicate zero elements. As illustrated in FIG. 2, the non-zero elements of a row of A are multiplied with a corresponding row of B to form a corresponding row of C. When there is even one element that is non-zero in the row of A, all elements in the corresponding row of C are non-zero. As described above, in the matrix product of the matrix with sparse non-zero elements and the matrix with dense non-zero elements, the number of non-zero elements increases exponentially. For example, the number of elements that are non-zero in the intermediate tensor increases exponentially each time the mode product is calculated.

Therefore, the machine learning apparatus according to the embodiment converts the mode product of the label mode into a concatenation. The concatenation is to add an element of an element matrix to an element of a data tensor or an intermediate tensor, and is also referred to as an E operation. For the E operation, the matrix product (mode product) is also referred to as a P operation.

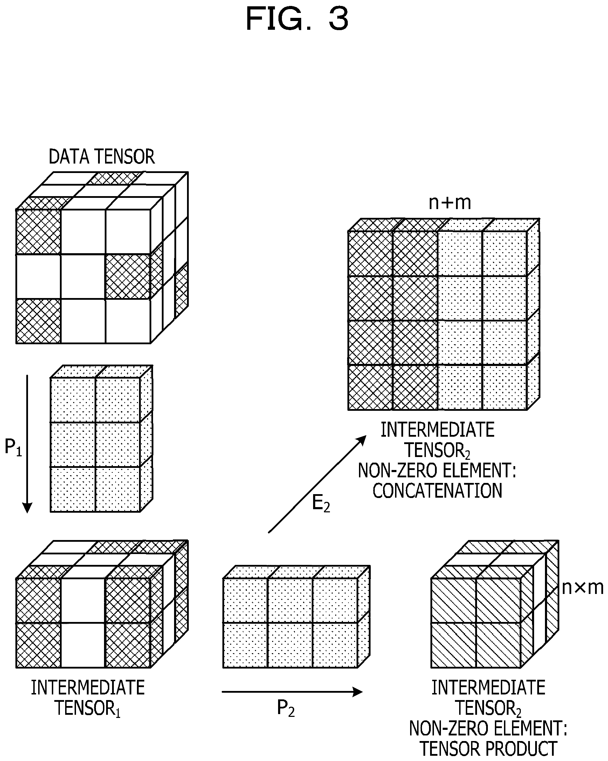

FIG. 3 is a diagram for explaining a difference in mode product and concatenation. FIG. 3 illustrates a difference between a mode product $P_2$ and a concatenation $E_2$. In FIG. 3, a shaded cube indicates a non-zero element, and a white cube indicates a zero element. n is a size of a non-zero element related to a mode "2" of an intermediate tensor 1, and m is a size of a non-zero element of a column of an element matrix$_2$.

As illustrated in FIG. 3, in a case of the mode product $P_2$, the size of the non-zero element related to the mode "2" of the intermediate tensor$_2$ is n × m, whereas in a case of the concatenation $E_2$, the size of the non-zero element related to the mode "2" of the intermediate tensor$_2$ is n + m. For example, the machine learning apparatus according to the embodiment converts the mode product of the label mode to concatenation so that the number of non-zero elements linearly increases.

A tensor X having sparse non-zero elements is mixedly expressed by a pair (I, V) of an index table I and a vector V of non-zero tensor elements. In the mixed expression, a row of I corresponds to coordinates of an element of V, and a column of I corresponds to a mode of the tensor. For i-th row of I, the following equation indicates a sub-tensor unit $V_i$ having dense non-zero elements. In the mixed expression, zero elements are not expressed.

$$I_i = \left[ I_i^{(1)}, \cdots, I_i^{(n)} \right]$$

In the mixed expression, a mode product of X and the element matrix W is calculated in an order of index division, a tensor outer product, and a sum. For example, in the calculation of the mode product of the mode n, the index row is divided as in the following equation (1).

$$\left[ I_i^{(1)}, \cdots, I_i^{(n-1)} | I_i^{(n)} \right] = \left[ I\hat{~}_i | I_i^{(n)} \right] \qquad (1)$$

An output index table I' of the mode product is formed from I^$_i$. Each value of the output vector V' of the mode product is calculated by a sum of the outer products of the row of $V_i$ and the corresponding row of W as illustrated in the following equation (2). The calculation of the sum of equation (2) is performed for i satisfying the following equation (3).

$$V_j' = \sum_{i \in nnz(I_j')} V_i \otimes W_{I_i^{(n)}} \qquad (2)$$

$$nnz(I_j') = \left\{ i : \hat{I}_i = I_j' \right\} \qquad (3)$$

In the label mode, the calculation of the sum of equation (2) is not performed, and only the outer product is obtained. The machine learning apparatus according to the embodiment calculates the output vector of the concatenation by using the following equation (4).

$$\hat{V} = V \cup \left\{ \left[ W_{I_i^{(n)}} \right] \right\} \qquad (4)$$

In order to input a core tensor calculated by tensor decomposition to a neural network, the machine learning apparatus according to the embodiment creates an input to the neural network by flattening and concatenating elements of V. Therefore, the outer product of the mode products and the vector concatenation include the same information although the numbers of values are different from each other. Therefore, even when the mode product of the label mode is converted into concatenation, there is no influence on machine learning.

The machine learning apparatus according to the embodiment selects one axis from among axes of a plurality of specified topology modes. For example, since the topology mode expresses linking of data, there are the plurality of (at least one set (2)) having a link) topology modes. Therefore, the machine learning apparatus according to the embodiment selects one axis that may be a target of explanation from among the axes of the plurality of topology modes, for example, by accepting selection or the like by a user.

Next, when calculating the core tensor, the machine learning apparatus according to the embodiment performs the E operation on an axis of the label mode, and performs the P operation on an axis of the topology mode other than the selected one axis. When calculating the core tensor, the machine learning apparatus according to the embodiment performs, for one selected axis, the E operation of sharing a parameter (element of the element matrix) with the axis of the topology mode other than the axis. The E operation of sharing this parameter is an E operation of concatenating not only the parameter of one selected axis but also a parameter of the axis of the topology mode other than the axis.

Thus, an element corresponding to the one axis selected among the axes of the topology mode and elements corresponding to the axis of the topology mode other than the one axis are incorporated in the core tensor (core expression). For this reason, the core tensor (core expression) includes a data linking method (topology), and in the embodiment, by performing machine learning using such a core tensor, it is possible to easily grasp the fine linking method of the data.

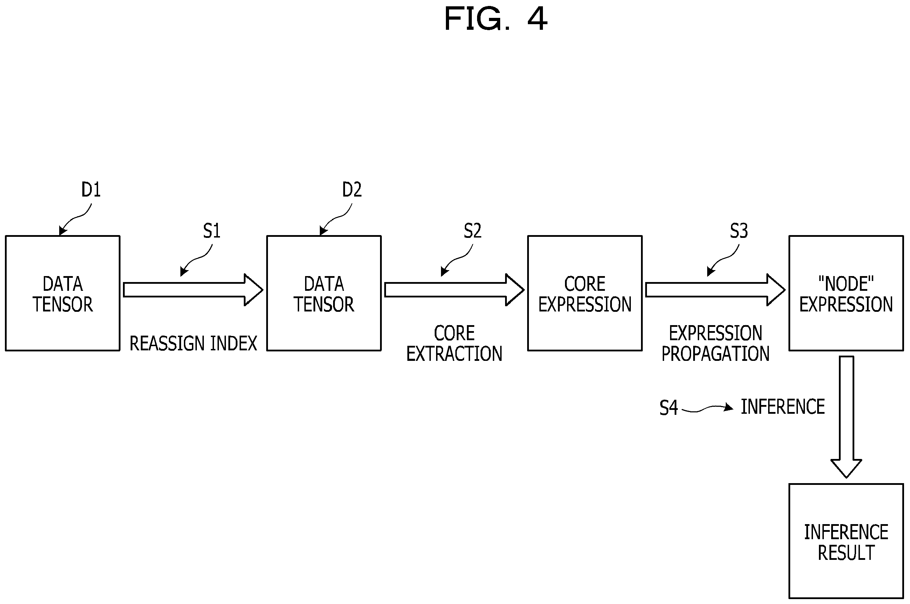

FIG. 4 is a diagram for explaining an overview of the embodiment. As illustrated in FIG. 4, the machine learning apparatus according to the embodiment reassigns the index a data tensor D1 in which a topology mode and a label mode are specified and one axis is selected among axes of the topology mode (step S1), and obtains a data tensor D2.

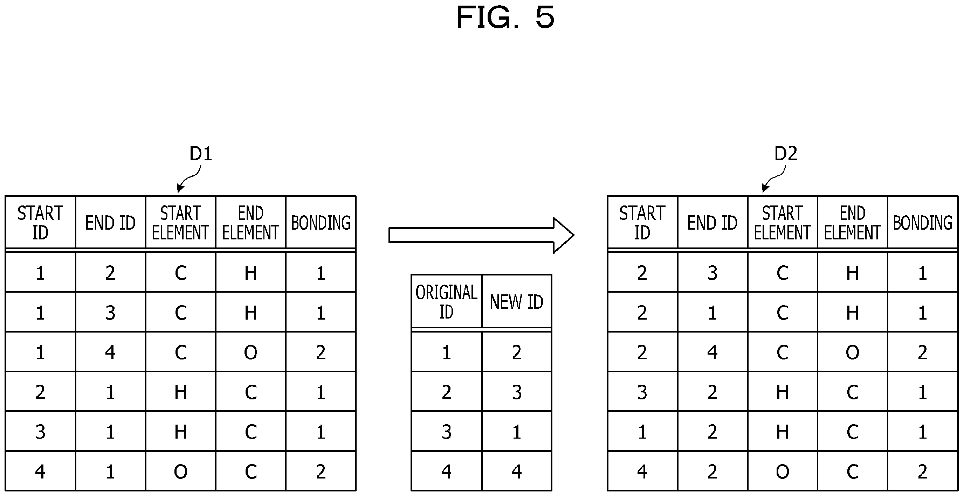

FIG. 5 is a diagram for explaining reassignment of an index. As illustrated in FIG. 5, the machine learning apparatus according to the embodiment generates the data tensor D2 by randomly reassigning a new element (index) to the data tensor D1 while maintaining information on a data linking method (topology). For example, the machine learning apparatus according to the embodiment determines an exchange table for replacing elements at random for each data and each learning epoch for an axis ("start ID" and "end ID" in the described example) of the topology mode.

Next, the machine learning apparatus according to the embodiment refers to the exchange table so that the same exchange table is used for IDs of the same group, and reassigns elements of the "start ID" and the "end ID" in the data tensor D1 to generate the data tensor D2. In the example in FIG. 5, an original ID "1" is reassigned to a new ID "2", an original ID "2" is reassigned to a new ID "3", an original ID "3" is reassigned to a new ID "1", and an original ID "4" is reassigned to a new ID "4".

Thus, in the machine learning using the data tensor D2, parameters of the machine learning model may be optimized so that the same inference result is obtained regardless of a value of the element.

Referring back to FIG. 4, the machine learning apparatus according to the embodiment calculates a core tensor (performs core extraction) by using the data tensor D2 (step S2). Specifically, the machine learning apparatus according to the embodiment performs the E operation on an axis of the label mode of the data tensor D2, and performs the P operation on an axis of the topology mode other than the selected axis. The machine learning apparatus according to the embodiment performs the E operation of sharing the parameter with the axis of the topology mode other than the axis, on the axis selected in the data tensor D2.

Next, the machine learning apparatus according to the embodiment executes machine learning of a machine learning model by using the calculated core tensor as an input (step S3, and step S4). For example, the machine learning apparatus according to the embodiment compares an inference result of the machine learning model inferred by information propagation (expression propagation) between nodes with a correct answer label assigned to the data tensor D1, and adjusts the parameter of the machine learning model.

A known method such as Multihead Self-Attention may be used for the information propagation. In a case of inference for each data (for example, toxicity of a compound expressed by a chemical formula), pooling (for example, Average Pooling) + Fully Convolutional Network (FCNN) may be used. In a case of the inference for each node (for example, health management, daily departure classification), FCNN may be used.

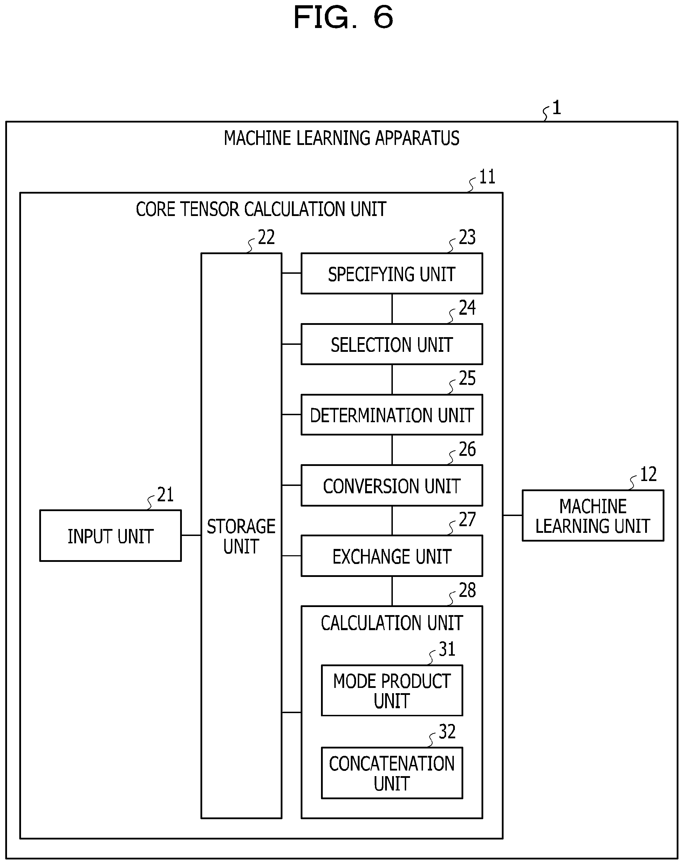

Next, a functional configuration of the machine learning apparatus according to the embodiment will be described. FIG. 6 illustrates a functional configuration example of the machine learning apparatus according to the embodiment. As illustrated in FIG. 6, a machine learning apparatus 1 according to the embodiment includes a core tensor calculation unit 11 and a machine learning unit 12.

The core tensor calculation unit 11 inputs information on the data tensor D1 and the number of elements of each mode of a core tensor to calculate the core tensor. The machine learning unit 12 performs machine learning by using the core tensor calculated by the core tensor calculation unit 11 to generate a machine learning model. The generated machine learning model is used for classification, prediction, and the like.

The core tensor calculation unit 11 includes an input unit 21, a storage unit 22, a specifying unit 23, a selection unit 24, a determination unit 25, a conversion unit 26, an exchange unit 27, and a calculation unit 28.

The input unit 21 inputs information on the data tensor D1 and the number of elements in each mode of the core tensor for storing in the storage unit 22.

The storage unit 22 stores the information input by the input unit 21. The storage unit 22 stores information created by the specifying unit 23, the selection unit 24, the determination unit 25, the conversion unit 26, the exchange unit 27, and the calculation unit 28.

The specifying unit 23 specifies whether each mode of the data tensor D1 is a topology mode or a label mode, and stores the specified result in the storage unit 22. For example, the specifying unit 23 specifies a target mode as the label mode in a case where a value of only one the target mode is 1 and the others are all 0 when values of all modes other than the target mode are fixed, and specifies the target mode as the topology mode in the other cases. Whether each mode of the data tensor is the topology mode or the label mode may be input by the input unit 21 and stored in the storage unit 22. Alternatively, the specifying unit 23 may specify the topology mode or the label mode by making an inquiry to the user.

The selection unit 24 selects one mode (axis) from the topology modes (axes) specified by the specifying unit 23, and stores the selection result in the storage unit 22. For example, the selection unit 24 makes an inquiry to the user, and stores the selection result input by the input unit 21 in the storage unit 22.

The determination unit 25 determines an order (execution order) of the modes of tensor decomposition so that the label mode is first and the topology mode is next, and stores the determined order in the storage unit 22. The determination unit 25 determines the order in which the topology mode selected by the selection unit 24 is last.

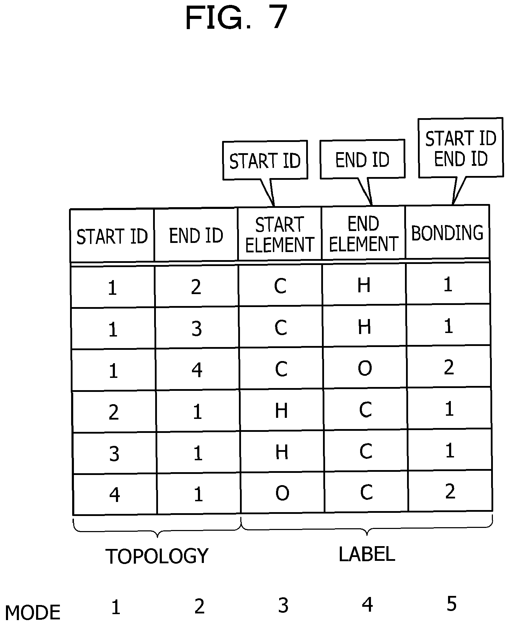

For example, as illustrated in FIG. 7, a mode of a start ID is "1", a mode of an end ID is "2", a mode of a start element is "3", a mode of an end element is "4", and a mode of bonding is "5". The mode of the start ID is set as the mode selected by the selection unit 24. In the example illustrated in FIG. 7, the determination unit 25 determines $P_5 P_4 P_3 P_2 P_1$ as the order of the tensor decomposition process. $P_5 P_4 P_3 P_2 P_1$ indicates that the tensor decomposition process is performed in the order of $P_5$, $P_4$, $P_3$, $P_2$, and Pi.

The conversion unit 26 converts the mode product P of the label mode into the concatenation E, and stores the conversion result in the storage unit 22. In the example illustrated in FIG. 7, since the mode "5", the mode "4", and the mode "3" are label modes, $P_5 P_4 P_3$ is converted into $E_5 E_4 E_3$, and an order of the tensor decomposition process is $E_5 E_4 E_3 P_2 P_1$.

For the topology mode, the conversion unit 26 reassigns the index described above, and stores the result obtained by randomly reassigned new elements in the storage unit 22.

For the topology mode selected by the selection unit 24, the conversion unit 26 converts the mode product P into the concatenation E of sharing parameters with other topology modes, and stores the conversion result in the storage unit 22. In the example illustrated in FIG. 7, the modes "1" and "2" are the label modes, and the mode "1" is the selected mode. Therefore, the conversion unit 26 converts Pi into $E_{1(P2)}$ that shares parameters with $P_2$. Thus, the order of the tensor decomposition processes is $E_5 E_4 E_3 P_2 E_{1(P2)}$.

The exchange unit 27 exchanges the order of the processes so as to process the concatenation E as late as possible, and stores the exchange result in the storage unit 22. Meanwhile, the conversion unit 26 has to perform the process of the label mode before the process of the topology mode associated with the label mode, and exchanges the order of the processes under a restriction that the concatenation E of sharing the parameters is the last.

For example, for $E_5 E_4 E_3 P_2 E_{1(P2)}$, in a case where $E_3$ is processed as late as possible, the mode "3" is associated with the mode "1", so that $E_3$ has to be performed before Pi. Therefore, $E_3$ and $P_2$ are exchanged, and $E_5 E_4 E_3 P_2 E_{1(P2)}$ becomes $E_5 E_4 P_2 E_3 E_{1(P2)}$. Since the mode "5" and the mode "4" are associated with the mode "2", further exchanging is not performed. By processing the concatenation E as late as possible, the core tensor calculation unit 11 may further reduce the number of non-zero elements of the intermediate tensor.

The exchange unit 27 specifies a topology mode associated with the label mode by, for example, making an inquiry to the user. The topology mode associated with the label mode may be input by the input unit 21, and stored in the storage unit 22.

The calculation unit 28 performs tensor decomposition based on the exchange result by the exchange unit 27 to calculate a core tensor. The calculation unit 28 stores information on the calculated core tensor in the storage unit 22. The calculation unit 28 includes a mode product unit 31, and a concatenation unit 32. The mode product unit 31 calculates a mode product (P operation) for a topology mode. The concatenation unit 32 performs concatenation calculation (E operation) for a label mode, and concatenation calculation (E operation) of sharing parameters for the selected mode.

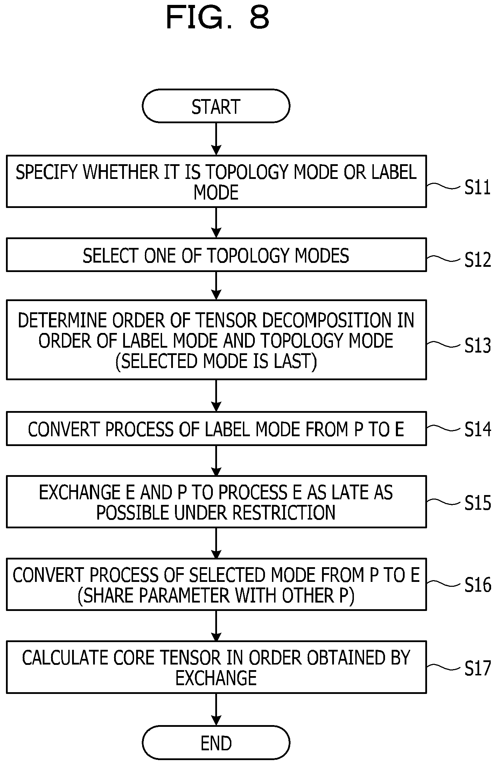

Next, a processing procedure by the core tensor calculation unit 11 will be described. FIG. 8 is a flowchart illustrating a procedure of a process by the core tensor calculation unit 11. For example, FIG. 8 illustrates a procedure of the process related to step S2 in FIG. 4.

As illustrated in FIG. 8, the core tensor calculation unit 11 specifies whether each mode of a data tensor is a topology mode or a label mode (step S11). Next, the core tensor calculation unit 11 selects one of the topology modes (step S12).

Next, the core tensor calculation unit 11 determines an order of tensor decomposition in an order of the label mode and the topology mode (step S13). The core tensor calculation unit 11 determines the mode selected in step S12 to be at an end of the order.

Next, the core tensor calculation unit 11 converts a process of the label mode from P to E (step S14). Next, the core tensor calculation unit 11 exchanges E and P so that E is processed as late as possible under a restriction that the process of the label mode is to be performed before a process of the topology mode associated with the label mode (step S15).

Next, the core tensor calculation unit 11 converts the process of the mode selected in step S12 from P to E (step S16). In this conversion, it is assumed that the core tensor calculation unit 11 shares a parameter with the other P. Next, the core tensor calculation unit 11 calculates a core tensor in the order obtained by the exchange (step S17).

As described above, in the embodiment, the specifying unit 23 specifies an axis of the label mode and an axis of the topology mode, among a plurality of axes included in data in a tensor format. The selection unit 24 selects one axis, among a plurality of specified axes of the topology mode. The calculation unit 28 calculates a core tensor from the data in the tensor format by a first process, a second process, and a third process. In the first process, an element included in an element matrix corresponding to an axis of a label mode is concatenated to an element included in an intermediate tensor. In the second process, a mode product of the intermediate tensor and an element matrix corresponding to an axis of the topology mode other than the selected one axis is calculated. In the third process, an element included in an element matrix corresponding to the selected one axis and the element included in the element matrix corresponding to the axis of the topology mode other than the axis are shared and concatenated to the element included in the intermediate tensor. The machine learning unit 12 executes machine learning of the machine learning model by using the core tensor calculated by the core tensor calculation unit 11 as an input.

Thus, in the core tensor (core expression), an element corresponding to one axis selected among axes of a topology mode and an element corresponding to an axis of the topology mode other than the one axis are incorporated, and a data linking method (topology) is included. Therefore, in the embodiment, by performing machine learning using such a core tensor, it is possible to easily grasp the fine linking method of data.

In the embodiment, the third process is executed at an end in the execution order, and thus a core tensor (core expression) in which the data linking method (topology) is incorporated last may be used for machine learning. Therefore, in the embodiment, it is possible to make it easier to grasp the fine linking method of data in machine learning.

It is noted that each of the components of each of the devices illustrated in the drawings is not necessarily physically configured as illustrated in the drawings. For example, specific forms of the separation and integration of each device are not limited to those illustrated in the drawings. The entirety or part of the device may be configured by functionally or physically separating into arbitrary units or integrating into an arbitrary unit in accordance with various loads, usage situations, and the like.

Although the machine learning apparatus 1 is described in the embodiment, a machine learning program having similar functions may be obtained by implementing the configuration of the machine learning apparatus 1 by software. A computer that executes the machine learning program will be described.

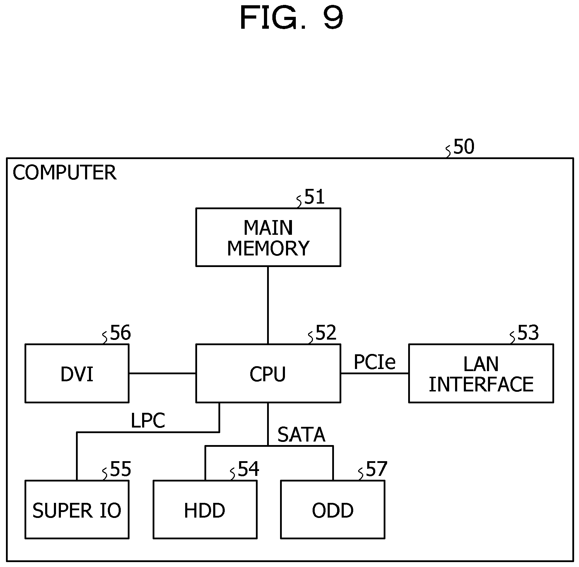

FIG. 9 is a diagram illustrating a hardware configuration of the computer that executes the machine learning program according to the embodiment. As illustrated in FIG. 9, a computer 50 includes a main memory 51, a central processing unit (CPU) 52, a local area network (LAN) interface 53, and a hard disk drive (HDD) 54. The computer 50 further includes a super input output (IO) 55, a digital visual interface (DVI) 56, and an optical disk drive (ODD) 57.

The main memory 51 is a memory that stores therein a program, a halfway result of program execution, and the like. The CPU 52 is a central processing unit that reads a program from the main memory 51, and executes the program, and is also an example of a control unit. The CPU 52 includes a chipset having a memory controller.

The LAN interface 53 is an interface that couples the computer 50 to another computer through the LAN. The HDD 54 is a disk device that stores a program and data, and the super IO 55 is an interface that couples input devices such as a mouse and a keyboard to the computer 50. The DVI 56 is an interface that couples a liquid crystal display device to the computer 50. The ODD 57 is a device that performs reading and writing of a Digital Versatile Disc (DVD) and a compact disc-recordable (CD-R).

The LAN interface 53 is coupled to the CPU 52 via Peripheral Component Interconnect Express (PCIe). The HDD 54 and the ODD 57 are coupled to the CPU 52 by Serial Advanced Technology Attachment (SATA). The super IO 55 is coupled to the CPU 52 by Low Pin Count (LPC).

The machine learning program executed by the computer 50 is stored on a CD-R which is an example of a recording medium that is readable by the computer 50, is read from the CD-R by the ODD 57, and is installed in the computer 50, for example. Alternatively, the machine learning program is stored in a database or the like of another computer system coupled via the LAN interface 53, is read out from the database or the like, and is installed in the computer 50. The installed machine learning program is stored in the HDD 54, is read out to the main memory 51, and is executed by the CPU 52.

Regarding the foregoing embodiment, the following appendices are further disclosed.

**Claims**

1. A machine learning program that causes at least one computer to execute a process, the process comprising:

   specifying an axis of a label mode and a plurality of axes of a topology mode among a plurality of axes included in data in a tensor format;

selecting a certain axis among the plurality axes of the topology mode;
calculating a core tensor from the data in the tensor format via a plurality of intermediate tensors, by a first process of concatenating an element included in a first element matrix corresponding to the axis of the label mode to an element included in a first intermediate tensor among the plurality of intermediate tensors, by a second process of calculating a mode product of a second intermediate tensor among the plurality of intermediate tensors and a second element matrix corresponding to an axis among the plurality axes of the topology mode other than the certain axis, by a third process of concatenating an element included in a third element matrix corresponding to the certain axis and an element included in the second element matrix to an element included in a third intermediate tensor among the plurality of intermediate tensors; and
executing a machine learning by using the core tensor as an input.

2. The machine learning program according to claim 1, wherein the process further comprising

determining an execution order of the first process, the second process, and the third process, wherein
the calculating includes executing the first process, the second process, and the third process in the order.

3. The machine learning program according to claim 2, wherein the determining includes determining the third process to be at an end of the order.

4. A machine learning method for a computer to execute a process comprising:

specifying an axis of a label mode and a plurality of axes of a topology mode among a plurality of axes included in data in a tensor format;
selecting a certain axis among the plurality axes of the topology mode;
calculating a core tensor from the data in the tensor format via a plurality of intermediate tensors, by a first process of concatenating an element included in a first element matrix corresponding to the axis of the label mode to an element included in a first intermediate tensor among the plurality of intermediate tensors, by a second process of calculating a mode product of a second intermediate tensor among the plurality of intermediate tensors and a second element matrix corresponding to an axis among the plurality axes of the topology mode other than the certain axis, by a third process of concatenating an element included in a third element matrix corresponding to the certain axis and an element included in the second element matrix to an element included in a third intermediate tensor among the plurality of intermediate tensors; and
executing a machine learning by using the core tensor as an input.

5. The machine learning method according to claim 4, wherein the process further comprising

determining an execution order of the first process, the second process, and the third process, wherein
the calculating includes executing the first process, the second process, and the third process in the order.

6. The machine learning method according to claim 5,
wherein the determining includes determining the third process to be at an end of the order.

7. A machine learning apparatus device comprising a control unit configured to:

specify an axis of a label mode and a plurality of axes of a topology mode among a plurality of axes included in data in a tensor format,
select a certain axis among the plurality axes of the topology mode,
calculate a core tensor from the data in the tensor format via a plurality of intermediate tensors, by a first process of concatenating an element included in a first element matrix corresponding to the axis of the label mode to an element included in a first intermediate tensor among the plurality of intermediate tensors, by a second process of calculating a mode product of a second intermediate tensor among the plurality of intermediate tensors and a second element matrix corresponding to an axis among the plurality axes of the topology mode other than the certain axis, by a third process of concatenating an element included in a third element matrix corresponding to the certain axis and an element included in the second element matrix to an element included in a third intermediate tensor among the plurality of intermediate tensors, and
execute a machine learning by using the core tensor as an input.

8. The machine learning device according to claim 7, wherein the control unit is further configured to:

determine an execution order of the first process, the second process, and the third process, and execute the first process, the second process, and the third process in the order.

9. The machine learning device according to claim 8,
   wherein the control unit is further configured to determine the third process to be at an end of the order.

# FIG. 1

|  |  | START ID | END ID | START ID / END ID |
|:---:|:---:|:---:|:---:|:---:|
| START ID | END ID | START ELEMENT | END ELEMENT | BONDING |
| 1 | 2 | C | H | 1 |
| 1 | 3 | C | H | 1 |
| 1 | 4 | C | O | 2 |
| 2 | 1 | H | C | 1 |
| 3 | 1 | H | C | 1 |
| 4 | 1 | O | C | 2 |

TOPOLOGY　　　　　LABEL

# FIG. 2

A   ×   B   =   C

# FIG. 3

DATA TENSOR

$P_1$

INTERMEDIATE
TENSOR$_1$

$P_2$

$E_2$

n+m

INTERMEDIATE
TENSOR$_2$
NON-ZERO ELEMENT:
CONCATENATION

n×m

INTERMEDIATE
TENSOR$_2$
NON-ZERO ELEMENT:
TENSOR PRODUCT

# FIG. 4

| D1 | | S1 REASSIGN INDEX | D2 | | S2 CORE EXTRACTION | | S3 EXPRESSION PROPAGATION | | S4 → INFERENCE | |
|---|---|---|---|---|---|---|---|---|---|---|

DATA TENSOR → DATA TENSOR → CORE EXPRESSION → "NODE" EXPRESSION → INFERENCE RESULT

# FIG. 5

D1

| START ID | END ID | START ELEMENT | END ELEMENT | BONDING |
|---|---|---|---|---|
| 1 | 2 | C | H | 1 |
| 1 | 3 | C | H | 1 |
| 1 | 4 | C | O | 2 |
| 2 | 1 | H | C | 1 |
| 3 | 1 | H | C | 1 |
| 4 | 1 | O | C | 2 |

| ORIGINAL ID | NEW ID |
|---|---|
| 1 | 2 |
| 2 | 3 |
| 3 | 1 |
| 4 | 4 |

D2

| START ID | END ID | START ELEMENT | END ELEMENT | BONDING |
|---|---|---|---|---|
| 2 | 3 | C | H | 1 |
| 2 | 1 | C | H | 1 |
| 2 | 4 | C | O | 2 |
| 3 | 2 | H | C | 1 |
| 1 | 2 | H | C | 1 |
| 4 | 2 | O | C | 2 |

EP 4 036 764 A1

# FIG. 6

# FIG. 7

| START ID | END ID | START ELEMENT | END ELEMENT | BONDING |
|----------|--------|---------------|-------------|---------|
| 1 | 2 | C | H | 1 |
| 1 | 3 | C | H | 1 |
| 1 | 4 | C | O | 2 |
| 2 | 1 | H | C | 1 |
| 3 | 1 | H | C | 1 |
| 4 | 1 | O | C | 2 |

START ID

END ID

START ID
END ID

TOPOLOGY               LABEL

MODE          1          2          3          4          5

# FIG. 8

START

↓

SPECIFY WHETHER IT IS TOPOLOGY MODE OR LABEL MODE — S11

↓

SELECT ONE OF TOPOLOGY MODES — S12

↓

DETERMINE ORDER OF TENSOR DECOMPOSITION IN ORDER OF LABEL MODE AND TOPOLOGY MODE (SELECTED MODE IS LAST) — S13

↓

CONVERT PROCESS OF LABEL MODE FROM P TO E — S14

↓

EXCHANGE E AND P TO PROCESS E AS LATE AS POSSIBLE UNDER RESTRICTION — S15

↓

CONVERT PROCESS OF SELECTED MODE FROM P TO E (SHARE PARAMETER WITH OTHER P) — S16

↓

CALCULATE CORE TENSOR IN ORDER OBTAINED BY EXCHANGE — S17

↓

END

# FIG. 9

**COMPUTER** — 50

**MAIN MEMORY** — 51

**DVI** — 56

**CPU** — 52

**LAN INTERFACE** — 53

PCIe

LPC

SATA

**SUPER IO** — 55

**HDD** — 54

**ODD** — 57

# FIG. 10A

```
      H
      |
      C = O
      |
      H
```

# FIG. 10B

| START ID | END ID | START ELEMENT | END ELEMENT | BONDING | VALUE |
|----------|--------|---------------|-------------|---------|-------|
| 1 | 2 | C | H | 1 | 1 |
| 1 | 3 | C | H | 1 | 1 |
| 1 | 4 | C | O | 2 | 1 |
| 2 | 1 | H | C | 1 | 1 |
| 3 | 1 | H | C | 1 | 1 |
| 4 | 1 | O | C | 2 | 1 |

# FIG. 11

DATA TENSOR

ELEMENT MATRIX

CORE TENSOR

$\approx$

## FIG. 12A

## FIG. 12B

DATA TENSOR

CORE TENSOR

ELEMENT
MATRIX

$P_1$

$P_3$

INTERMEDIATE
TENSOR$_1$

$P_2$

INTERMEDIATE
TENSOR$_2$

Europäisches
Patentamt

European
Patent Office

Office européen
des brevets

# EUROPEAN SEARCH REPORT

Application Number

EP 21 21 3112

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MO YU ET AL: "Embedding Lexical Features via Low-Rank Tensors", ARXIV.ORG, CORNELL UNIVERSITY LIBRARY, 201 OLIN LIBRARY CORNELL UNIVERSITY ITHACA, NY 14853, 2 April 2016 (2016-04-02), XP080692927, * sections 3 and 4 * | 1-9 | INV. G06F17/16 G06N20/00 |
| A | ALP OZDEMIR ET AL: "Multiscale Analysis for Higher-order Tensors", ARXIV.ORG, CORNELL UNIVERSITY LIBRARY, 201 OLIN LIBRARY CORNELL UNIVERSITY ITHACA, NY 14853, 27 April 2017 (2017-04-27), XP080766031, * sections II and III * | 1-9 | |

TECHNICAL FIELDS
SEARCHED (IPC)

G06F

The present search report has been drawn up for all claims

| Place of search | Date of completion of the search | Examiner |
|---|---|---|
| Berlin | 19 May 2022 | Virnik, Elena |

EPO FORM 1503 03.82 (P04C01)

**REFERENCES CITED IN THE DESCRIPTION**

**Patent documents cited in the description**

- JP 2020119101 A
- US 20190228304
- US 20190325312